# EUROPÄISCHE PATENTSCHRIFT

(11) **EP 0 859 747 B1**
(45) Veröffentlichungstag und Bekanntmachung des Hinweises auf die Patenterteilung: **10.10.2001**
(21) Anmeldenummer: 97924858.0
(22) Anmeldetag: 11.03.1997
(51) Int. Cl.: C02F 3/34, C12N 1/20

(54) **VERFAHREN ZUM AEROBEN BIOLOGISCHEN ABBAU SCHWER WASSERLÖSLICHER STOFFE SOWIE MIKROORGANISMUS STAMM IHI-91**
PROCESS FOR THE AEROBIC BIOLOGICAL BREAK-DOWN OF SUBSTANCES HAVING LOW WATER-SOLUBILITY, AND A MICRO-ORGANISM OF THE STRAIN IHI-91
PROCEDE POUR LA BIODEGRADATION AEROBIE DE MATIERES DIFFICILEMENT HYDROSOLUBLES ET MICROORGANISME SOUCHE IHI-91

(30) Priorität: 12.03.1996 DE 19609555
(43) Veröffentlichungstag der Anmeldung: 26.08.1998
(73) Patentinhaber: Märkl, Herbert, Prof. Dr.-Ing., 21218 Seevetal (DE); Antranikian, Garabed, Prof. Dr., 21218 Seevetal (DE)
(72) Erfinder: MÄRKL, Herbert, 21218 Seevetal (DE); ANTRANIKIAN, Garabed, 21218 Seevetal (DE); BECKER, Peter, 20255 Hamburg (DE); MARKOSSIAN, Samson, 21075 Hamburg (DE)
(74) Vertreter: Pohl, Manfred, Dr.
(86) Internationale Anmeldenummer: DE9700461
(87) Internationale Veröffentlichungsnummer: WO9733838

(56) Entgegenhaltungen:
- EP-A- 0 735 005
- DECHEMA MONOGR., Bd. 133, 1996, Seiten 615-622, XP002036570 HEBENBROCK,S.: "Biodegradation of aromatic and aliphatic hydrocarbons by Thermophilic microorganisms and their cultivation in bioreactors."
- BIOTECHNOLOGY LETTERS, Bd. 15, Nr. 4, April 1993, MANCHESTER UK, Seiten 361-6, XP002036571 SIGURG SLAD TTIR,S.: "Lipase activity of thermophilic bacteria from icelandic hot springs."
- JOURNAL OF FERMENTATION AND BIOENGINEERING, Bd. 79, Nr. 5, 1995, JAPAN, Seiten 433-8, XP002036572 WANG,Y.: "Thermostable Alcaline Lipase from a newly isolated thermophilic bacillus, strain A30-1."

## Beschreibung

Die Erfindung betrifft ein Verfahren zum aeroben biologischen Abbau schwer wasserlöslicher Stoffe in einem wässrigen Medium sowie einen neuen Mikroorganismus.

Verfahren zum aeroben biologischen Abbau sind allgemein bekannt. Beispielsweise findet bei der Abwasserreinigung ein biologischer Abbau verschiedener Stoffe unter aeroben Bedingungen statt, wobei die meisten löslichen Stoffe gut umgesetzt werden. Bei schwer wasserlöslichen Stoffen besteht jedoch das Problem, daß diese zum biologischen Abbau zunächst in die wäßrige Phase überführt werden müssen, bevor sie von Organismen, wie z.B. Bakterien, aufgenommen und umgesetzt werden können.

Um die Löslichkeit der abzubauenden Stoffe zu verbessern, können Lösungsmittel eingesetzt werden, die die Abbaubarkeit der Stoffe ermöglichen. Bei den Lösungsmitteln handelt es sich häufig um organische Verbindungen, die unter Umständen eine große Belastung für die Umwelt darstellen. Insofern müssen die Lösungsmittel ebenfalls abbaubar sein, ohne daß toxische Rückstände verbleiben und die Gesamtbelastung steigt.

Eine weitere Möglichkeit, den Abbau schwer wasserlöslicher Stoffe zu verbessern, besteht in einer Erhöhung der Temperatur. Aus EP 0735005 ist ein Verfahren zum biologischen Abbau organischer Verbindungen bei Temperaturen von 45 bis 85 °C bekannt, wobei auch sogenannte "hot cells" beschrieben sind. Bei diesen "hot cells" handelt es sich jedoch um adaptierte Mikroorganismen, die keine zufriedenstellenden Abbauleistungen zeigen. Dies gilt insbesondere für Verbindungen, die schlecht wasserlöslich sind.

Aufgabe der vorliegenden Erfindung ist es daher, den Einsatz von Lösungsmitteln zu vermeiden und ein umweltverträgliches, gleichzeitig aber effizientes biologisches Verfahren zu schaffen, mit dem ein Abbau der schwer wasserlöslichen Stoffe möglich ist.

Erfindungsgemäß erfolgt die Lösung der gestellten Aufgabe dadurch, daß man die Bioverfügbarkeit der abzubauenden Stoffe steigert, indem man die Temperatur des wässrigen Mediums auf Werte von 45 °C und darüber einstellt, und daß man zum Abbau der schwer wasserlöslichen Stoffe eine Kultur thermophiler Mikroorganismen verwendet, die aerob wächst und den Mikroorganismus Stamm IHI-91 (hinterlegt bei der DSMZ unter der Nummer DSM 10561) enthält.

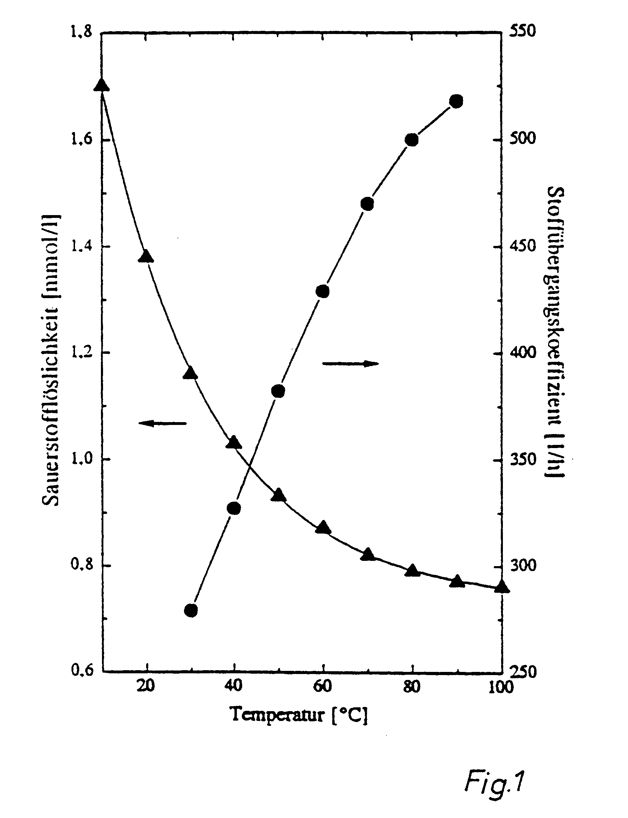

Der vorliegenden Erfindung liegt die überraschende Erkenntnis zugrunde, daß mit steigender Temperatur nicht nur die Bioverfügbarkeit der abzubauenden Stoffe steigt, sondern daß sich gleichzeitig auch der Sauerstoffübergangskoeffizient erhöht. Diese Erkenntnis wurde von der Fachwelt bislang nicht wahrgenommen. Vielmehr ist man stets davon ausgegangen, daß bei höheren Temperaturen kein effizienter biologischer Abbau unter aeroben Bedingungen stattfinden kann, weil sich die Sauerstofflöslichkeit zu sehr verringert. Wie Abbildung 1 zeigt, wird die Abnahme der Sauerstofflöslichkeit durch den Anstieg des Sauerstoffübergangskoeffizienten kompensiert. Insofern ist es möglich, auch bei höheren Temperaturen thermophile Mikroorganismen einzusetzen und diese so mit Sauerstoff zu versorgen, daß sie aerob wachsen können. Die Belüftung kann hierbei in üblicher Weise mittels Luft oder reinem Sauerstoff erfolgen.

Nach einer besonderen Ausgestaltung des Verfahrens erhöht man die Temperatur auf Werte über 60 °C. In diesem Temperaturbereich wachsen thermophile Mikroorganismen, die insbesondere zum Abbau schwer wasserlöslicher Stoffe einsetzbar sind. Ebenso hat der neue Stamm IHI-91 ein Temperaturoptimum im Bereich von 65 °C. Je nach Temperaturoptimum der verwendeten Kultur kann das Verfahren z.B. auch bei höheren Temperaturen durchgeführt werden.

Das erfindungsgemäße Verfahren läßt sich insbesondere zum Abbau schwer wasserlöslicher Fette und fettartiger Verbindungen einsetzen. Diese Verbindungen sollen insbesondere alle diejenigen Stoffe umfassen, die enzymatisch mittels Lipasen spaltbar sind. Allerdings ist das erfindungsgemäße Verfahren nicht auf diese Verbindungen beschränkt, sondern läßt sich im Grunde für alle schwer wasserlöslichen Stoffe einsetzen, deren Löslichkeit mit zunehmender Temperatur steigt und die mittels thermophiler Mikroorganismen aerob abbaubar sind. Beispielsweise auch für aromatische Kohlenwasserstoffe und insbesondere für polycyclische aromatische Kohlenwasserstoffe.

Folgende Tabelle gibt eine Auswahl aus dem breiten Substratspektrum für aerobe thermophile Mikroorganismen. Diese Stoffe lassen sich durch Anwendung des erfindungsgemäßen Verfahrens problemlos abbauen.

| **Substrat** | **Vorkommen/Verwendung** |
|---|---|
| Tween 80 | Emulgator |
| Silikonöl(Dimethylpolysiloxan) | Anti-Schaum-Mittel |
| Olivenöl, Triolein | Speiseölherstellung |
| Tributyrin | Weichmacher |
| Palmitin-, Stearinsäure | Seifenherstellung |
| Glycerin | vielfältige Verwendung |
| Lanolin (Wollfett) | Herstellung von Kosmetika |

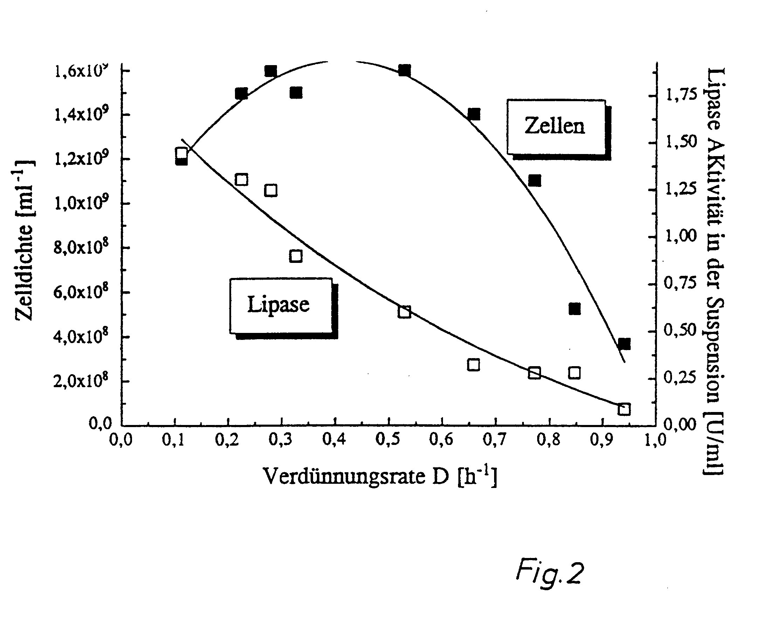

Das erfindungsgemäße Verfahren verwendet den Mikroorganismus Stamm IHI-91 (hinterlegt bei der DSMZ-Deutsche Sammlung von Mikroorganismen und Zellkulturen GmbH, D-38124 Braunschweig, unter Nummer DSM 10561). Dieser Stamm zeichnet sich besonders durch seine Lipase-Aktivität aus. In einer kontinuierlichen Kultur dieses Stammes mit Olivenöl als Substrat wurden die reaktionskinetischen Daten K_{S} und µₘₐₓ ermittelt. Der K_{S}-Wert für Olivenöl beträgt 0,88 g/l. Die maximale Wachstumsrate µₘₐₓ wurde zu 0,94 h⁻¹ berechnet. Abbildung 2 zeigt das Ergebnis einer kontinuierlichen Fermentation dieses Stammes auf Olivenöl. Aufgetragen sind die Zelldichte und die Lipase-Aktivität als Funktion der Verdünnungsrate.

Aufgrund der besonderen Enzym-Aktivität des Stammes IHI-91 ist es auch möglich, aus dem Überstand der Kultur oder einem geeigneten Zellaufschluß eine Enzymzusammensetzung zu erhalten und diese zum Abbau von schwer wasserlöslichen Stoffen zu verwenden.

Das erfindungsgemäße Verfahren wird nachfolgend anhand eines ausgewählten Versuchsbeispiels beschrieben.

### Beispiel

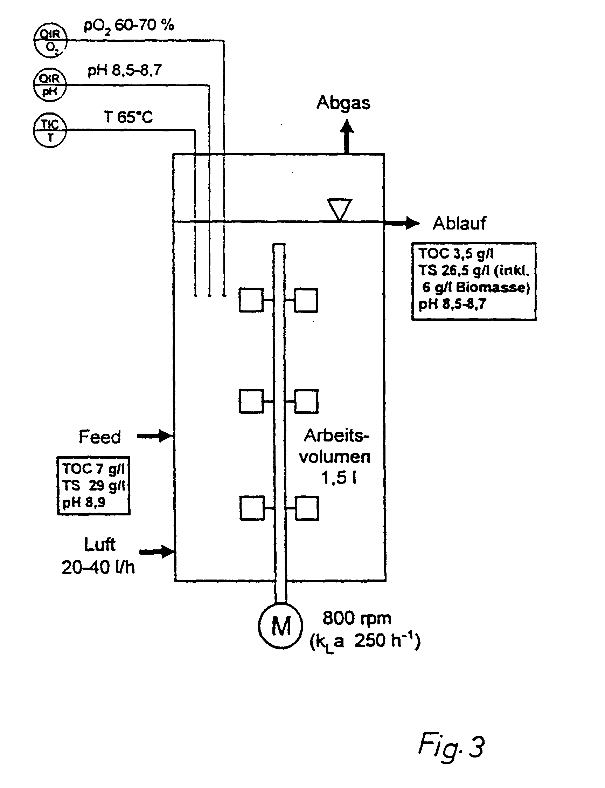

Bei diesem Versuch wurde industriell anfallendes Wollwaschwasser dem erfindungsgemäßen Verfahren unterzogen. Wollwaschwasser ist ein stark fetthaltiges Abwasser, das im industriellen Prozeß bei Temperaturen im Bereich von 50 bis 60 °C anfällt. Dieses Abwasser wurde in einen 2-Liter-Laborfermenter gefüllt und auf 65 °C erhitzt. Anschließend erfolgte eine Beimpfung mit dem neuen Stamm IHI-91 bis zu einer Zelldichte von 10⁹ Zellen/ml. Abbildung 3 zeigt den entsprechenden Versuchsaufbau. Nach einer anfänglichen Batch-Phase von etwa 24 h wurde auf einen kontinuierlichen Betrieb mit einer Verweilzeit von 20 h übergegangen. Während eines sechstägigen Betriebs wurden der Feststoffgehalt (TS), der TOC-Gehalt (Total Organic Carbon), die Lipase-Aktivität, der pH-Wert, der Sauerstoffverbrauch und die Zelldichte ermittelt. Bereits nach kurzer Zeit etablierte sich eine stabile Biomasse mit einer Organismendichte von etwa 10¹⁰ Zellen/ml, entsprechend einer Zelltrockensubstanz von ca. 6 g/l. Während des kontinuierlichen Betriebs wurde eine TOC-Abnahme um konstant 50% gemessen, wobei sich ein Sauerstoffverbrauch von etwa 1 g O₂/l und Stunde einstellte. Zugleich war eine deutliche Reduzierung der sich oben abscheidenden Festphase zu beobachten. Über den gesamten Versuchszeitraum konnte eine hohe Aktivität einer thermophilen Lipase nachgewiesen werden, die für die Fetthydrolyse verantwortlich ist.

### Stammcharakterisierung (IHI-91):

Der neue Mikroorganismus Stamm IHI-91 (hinterlegt bei der DSMZ-Deutsche Sammlung von Mikroorganismen und Zellkulturen GmbH, D-38124 Braunschweig, unter der Nummer DSM 10561) läßt sich wie folgt charakterisieren:

Die partielle Sequenzierung der 16SrDNA ergab eine eindeutige Zuordnung dieses Stammes zu der Gruppe 5 der Gattung *Bacillus* (Ash et al., Letters in Appl. Microbiol. 13, 202-206, 1991). Es wurde eine Ähnlichkeit von 99,8% zu den Arten *Bacillus kaustophilus*, *Bacillus thermoleovorans*, *Bacillus caldotenax* und *Bacillus caldolyticus* festgestellt.

Aufgrund der physiologischen Merkmale ist der Mikroorganismus Stamm IHI-91 der Species *Bacillus thermoleovorans* am ähnlichsten. Diese Zuordnung konnte durch die DNA/DNA-Hybridisierung mit einem Wert von 82,5% bestätigt werden.

Eigenschaften des Stammes IHI-91:

| Zellform | Stäbchen |
|---|---|
| Breite µm | 0,7-0,8 |
| Länge µm | 2,5-5,0 |
| | |

| Sporen | |
|---|---|
| ellipsoid | + |
| rund | - |
| geschwollen | d |
| | |
| Catalase | + |
| | |
| Anaerobes Wachstum | - |
| | |
| VP Reaktion | k.W. |
| | |

| Optimale Temperatur | |
|---|---|
| Wachstum positiv bei °C | 65 |
| Wachstum negativ bei <°C | 45 |
| | |

| Säure aus | |
|---|---|
| D-Glucose | + |
| L-Arabinose | + |
| D-Xylose | - |
| D-Mannit | + |
| D-Fructose | + |
| | |
| Gas aus Glucose | - |
| | |
| Lecithinase | k.W. |
| | |

| Hydrolyse von | |
|---|---|
| Stärke | + |
| Gelatine | + |
| Casein | + |
| Tween 80 | + |
| Äskulin | - |
| | |

| Verwertung von | |
|---|---|
| Citrat nach Koser | + |
| Propionat | + |
| | |
| Abbau von Tyrosin | - |
| | |
| NO₂⁻ aus NO₃⁻ | + |
| | |
| Indol | - |
| | |
| Phenylalanindesaminase | - |
| | |
| Arginindihydrolase | - |
| | |
| Urease | + |
| | |

| Abkürzungen: | |
|---|---|
| k.W. | kein Wachstum |
| d | unterschiedlich |

### Fettsäureprofil:

Die charakteristischen Fettsäuren für die Gattung *Bacillus* konnten nachgewiesen werden.

## Patentansprüche

1. Verfahren zum aeroben biologischen Abbau schwer wasserlöslicher Stoffe in einem wässrigen Medium, **dadurch gekennzeichnet, daß** man die Bioverfügbarkeit der abzubauenden Stoffe steigert, indem man die Temperatur des wässrigen Mediums auf Werte von 45 °C und darüber einstellt, und daß man zum Abbau der schwer wasserlöslichen Stoffe eine Kultur thermophiler Mikroorganismen verwendet, die aerob wächst und den Mikroorganismus Stamm IHI-91 (hinterlegt bei der DSMZ-Deutsche Sammlung von Mikroorganismen und Zellkulturen GmbH, D-38124 Braunschweig, unter der Nummer DSM 10561) enthält.

2. Verfahren nach Anspruch 1, **dadurch gekennzeichnet, daß** man die Temperatur auf Werte über 60 °C erhöht.

3. Verfahren nach Anspruch 1 oder 2, **dadurch gekennzeichnet, daß** man als schwer wasserlösliche Stoffe Fette und fettartige Verbindungen einsetzt.

4. Verfahren nach Anspruch 1 oder 2, **dadurch gekennzeichnet, daß** man als schwer wasserlösliche Stoffe aromatische Kohlenwasserstoffe, insbesondere polycyclische aromatische Kohlenwasserstoffe, einsetzt.

5. Mikroorganismus Stamm IHI-91 (hinterlegt bei der DSMZ-Deutsche Sammlung von Mikroorganismen und Zellkulturen GmbH, D-38124 Braunschweig, unter der Nummer DSM 10561).

6. Enzymzusammensetzung erhältlich aus dem Mikroorganismus nach Anspruch 5 zum biologischen Abbau schwer wasserlöslicher Stoffe.

7. Enzymzusammensetzung nach Anspruch 6, **dadurch gekennzeichnet, daß** die Enzymzusammensetzung Lipase-Aktivität aufweist.

## Claims

1. A process for the aerobic biodegradation in an aqueous medium of compounds having a low water-solubility, wherein the bioavailability of the compounds to be degraded is increased by setting the aqueous medium to temperature values of 45 °C and higher; and
a culture of thermophilic microorganisms is used that grows aerobically and comprises a microorganism of the strain IHI-91 (deposited with the DSMZ-Deutsche Sammlung von Mikroorganismen und Zellkulturen GmbH, D-38124 Braunschweig and having accession number DSM 10561).

2. Process according to claim 1, wherein the temperature is increased to values above 60 °C.

3. Process according to claim 1 or 2, wherein fats and fatty compounds are used as substances having low water-solubility.

4. Process according to claim 1 or 2, wherein aromatic hydrocarbons, especially polycyclic aromatic hydrocarbons, are used as substances having low water-solubility.

5. A microorganism strain IHI-91 (deposited with the DSMZ-Deutsche Sammlung von Mikroorganismen und Zellkulturen GmbH, D-38124 Braunschweig and having accession number DSM 10561).

6. An enzyme composition obtainable from the microorganism according to claim 5 for the biodegradation of compounds having low water-solubility.

7. Enzyme composition according to claim 6, wherein the enzyme composition demonstrates lipase activity.

## Revendications

1. Procédé de biodégradation aérobie de matières difficilement solubles dans l'eau contenues dans un milieu aqueux, **caractérisé en ce que** l'on augmente la biodisponibilité des matières à dégrader en réglant la température du milieu aqueux à une valeur de 45 °C ou plus et **en ce que** l'on utilise pour la dégradation des matières difficilement solubles dans l'eau une culture de micro-organismes thermophiles qui croît de façon aérobie et qui contient la souche de micro-organisme IHI-91 (déposée auprès de DSMZ-Deutsche Sammlung von Mikroorganismen und Zellkulturen GmbH, D-38124 Braunschweig, sous le numéro DSM 10561).

2. Procédé selon la revendication 1, **caractérisé en ce que** l'on augmente la température à des valeurs supérieures à 60 °C.

3. Procédé selon la revendication 1 ou 2, **caractérisé en ce que** l'on utilise comme substances difficilement solubles dans l'eau des graisses et des composés de type graisseux.

4. Procédé selon la revendication 1 ou 2, **caractérisé en ce que** l'on utilise comme substances difficilement solubles dans l'eau des hydrocarbures aromatiques, notamment des hydrocarbures aromatiques polycycliques.

5. Souche de microorganismes IHI-91 (déposée auprès de DSMZ-Deutsche Sammlung von Mikroorganismen und Zellkulturen GmbH, D-38124 Braunschweig, sous le numéro DSM 10561).

6. Composition enzymatique pouvant être obtenue à partir du micro-organisme selon la revendication 5 pour la biodegradation de matières difficilement solubles dans l'eau.

7. Composition enzymatique selon la revendication 6, **caractérisé en ce que** la composition enzymatique a une activité lipasique.
